# FASCICULE DE BREVET EUROPEEN

(11) **EP 4 009 878 B1**
(45) Date de publication et mention de la délivrance du brevet: **28.02.2024**
(21) Numéro de dépôt: 20753348.0
(22) Date de dépôt: 06.08.2020
(51) Int. Cl.: A61B 10/02, A61B 17/34, A61B 90/50, A61B 8/00, A61N 5/00

(54) **DISPOSITIF DE MAINTIEN D'UN INSTRUMENT MEDICAL ET INSTALLATION COMPRENANT UN TEL DISPOSITIF**
VORRICHTUNG ZUM HALTEN EINES MEDIZINISCHEN INSTRUMENTS UND EINRICHTUNG MIT SOLCH EINER VORRICHTUNG
DEVICE FOR HOLDING A MEDICAL INSTRUMENT AND APPARATUS COMPRISING SUCH A DEVICE

(30) Priorité: 08.08.2019 FR 1909085
(43) Date de publication de la demande: 15.06.2022
(73) Titulaire: Koelis, 38240 Meylan (FR)
(72) Inventeur: LEROY, Antoine, 78390 Bois d`Arcy (FR); BRONSARD, Tristan, 38660 Le Touvet (FR)
(74) Mandataire: Cabinet Boettcher
(86) Numéro de dépôt international: PCT/EP2020/072184
(87) Numéro de publication internationale: WO 2021/023838

(56) Documents cités:
- US-A- 4 838 506
- US-A- 5 494 039
- US-A- 5 976 092
- US-A1- 2002 007 103
- US-A1- 2011 288 541
- US-A1- 2017 151 033
- US-A1- 2019 231 386

## Description

L'invention concerne un dispositif de maintien d'un instrument médical.

L'invention concerne également une installation comprenant un tel dispositif.

### ARRIERE PLAN DE L'INVENTION

Grâce aux progrès des technologies employées et au bon suivi de la population à risque, les cancers de la prostate sont détectés de plus en plus tôt. En conséquence, les tumeurs décelées s'avèrent de plus en plus petites.

De la sorte les traitements classiques d'ablation totale ou partielle de la prostate ne sont aujourd'hui plus adaptés en particulier en regard des graves conséquences post-opératoires qu'ils entraînent (incontinence, impuissance ...) .

Ainsi, les opérateurs se tournent de plus en plus vers des traitements ciblés consistant à insérer un instrument dans le corps du patient pour approcher l'instrument d'une zone particulière de la prostate à traiter. Les traitements sont par exemple une curiethérapie avec l'insertion d'un cathéter (pour le positionnement de grains radioactifs), ou encore d'autres thérapies ciblées telles que la cryothérapie ou la thérapie laser.

Un prérequis de ce type de traitements est que l'opérateur doit faire preuve d'une grande précision pour approcher correctement l'instrument de la zone visée. Or l'instrument doit parfois être maintenu en place plusieurs minutes à plusieurs dizaines de minutes afin que le traitement puisse être assuré de manière optimale ce qui est peu confortable pour l'opérateur. En outre, il lui est difficile de contrôler de petits mouvements involontaires de sa main dû à la position statique prolongée lors du traitement. Malheureusement ces mouvements non souhaités peuvent s'avérer dangereux pour le patient en particulier dans le cas où ils entraînent un enfoncement supplémentaire de l'instrument dans la prostate.

Un dispositif de guidage d'électrodes pour le traitement de tumeurs de la prostate est connu du document US2011/0288541A1.

### OBJET DE L'INVENTION

Un but de l'invention est de proposer un dispositif de maintien d'un instrument médical permettant de limiter un risque de déplacement involontaire de l'instrument lors du traitement d'une zone particulière ciblée du corps d'un patient à l'aide de l'instrument.

Un but de l'invention est également de proposer une installation comprenant un tel dispositif.

### RESUME DE L'INVENTION

En vue de la réalisation de ce but, on propose, selon l'invention, un dispositif de maintien d'un instrument médical, le dispositif comprenant :
- une base destinée à être portée par un appareil comprenant une sonde d'imagerie médicale associée à l'instrument médical, et
- une structure porteuse destinée à porter l'instrument médical et montée mobile sur la base de manière à pouvoir se déplacer sur la base entre une première position et une deuxième position, le dispositif étant apte à maintenir comme instrument médical un applicateur comprenant un manche à partir duquel s'étend une aiguille de traitement, la structure porteuse comprenant à cet effet une bague destinée à porter le manche de l'instrument médical.

L'invention permet ainsi de solidariser temporairement l'instrument médical au dispositif de maintien afin d'assister un opérateur dans son travail.

L'invention permet notamment de pouvoir maintenir fixe la position de l'instrument médical sans intervention de l'opérateur. On note en particulier que l'invention permet de maintenir fixe la profondeur avec laquelle l'instrument est éventuellement enfoncé dans un organe visé.

Néanmoins en cas de besoin l'instrument médical peut être retiré rapidement du dispositif de maintien.

De façon particulière, la bague n'est pas portée par un porte-aiguille du dispositif.

De façon particulière, la structure porteuse est montée coulissante sur la base.

De façon particulière, la structure porteuse comporte une colonne montée mobile sur la base et une bague montée mobile sur la colonne.

De façon particulière, la bague est montée coulissante sur la colonne.

De façon particulière, la bague est montée pivotante sur la colonne.

De façon particulière, la bague comporte des moyens de fixation temporaire de l'instrument au dispositif.

De façon particulière, la bague comporte au moins une fenêtre pour visualiser la colonne.

De façon particulière, la bague comporte au moins un sigle de repérage de l'orientation relative de la bague vis-à-vis de la colonne.

De façon particulière, la colonne comporte au moins une succession de repères graphiques.

De façon particulière, la structure porteuse peut être montée sur la base selon au moins deux sens d'introduction différents.

De façon particulière, la structure porteuse comporte un élément de repérage pour chaque sens d'introduction possible de la structure porteuse sur la base.

De façon particulière, la base comporte au moins un signe de repérage pour symboliser où la base doit être montée en service sur l'appareil.

L'invention concerne également une installation comprenant le dispositif de maintien tel que précité et un guide-aiguille.

Optionnellement le guide-aiguille est une grille.

Optionnellement, l'installation comporte également l'appareil qui comprend une sonde échographique.

Optionnellement, la base et/ou la structure porteuse est une pièce moulée par injection.

Optionnellement, l'installation comprend l'instrument médical qui n'est pas un guide-aiguille.

D'autres caractéristiques et avantage de l'invention ressortiront à la lecture de la description qui suit de modes de mise en oeuvre particuliers non limitatifs de l'invention.

### BREVE DESCRIPTION DES DESSINS

L'invention sera mieux comprise à la lumière de la description qui suit en référence aux figures annexées parmi lesquelles :
[Fig. 1] la figure 1 illustre une installation selon un premier mode de réalisation de l'invention,
[Fig. 2] la figure 2 est une vue sensiblement de face du dispositif de l'installation illustrée à la figure 1, une bague du dispositif étant dans une première position,
[Fig. 3] la figure 3 est une vue similaire à celle de la figure 2, une bague du dispositif étant dans une deuxième position,
[Fig. 4a] la figure 4a illustre une première étape de fixation du dispositif de maintien à un appareil de l'installation illustrée à la figure 1,
[Fig. 4b] la figure 4b illustre une deuxième étape de fixation du dispositif de maintien à un appareil de l'installation illustrée à la figure 1,
[Fig. 4c] la figure 4c illustre une troisième étape de fixation du dispositif de maintien à un appareil de l'installation illustrée à la figure 1,
[Fig. 4d] la figure 4d illustre une quatrième étape de fixation du dispositif de maintien à un appareil de l'installation illustrée à la figure 1,
[Fig. 4e] la figure 4e illustre une cinquième étape de fixation du dispositif de maintien à un appareil de l'installation illustrée à la figure 1,
[Fig. 4f] la figure 4f illustre une sixième étape de fixation du dispositif de maintien à un appareil de l'installation illustrée à la figure 1,
[Fig. 5] la figure 5 illustre une installation selon un deuxième mode de réalisation de l'invention,
[Fig. 6] la figure 6 illustre une installation selon un troisième mode de réalisation de l'invention.

### DESCRIPTION DETAILLEE DE L'INVENTION

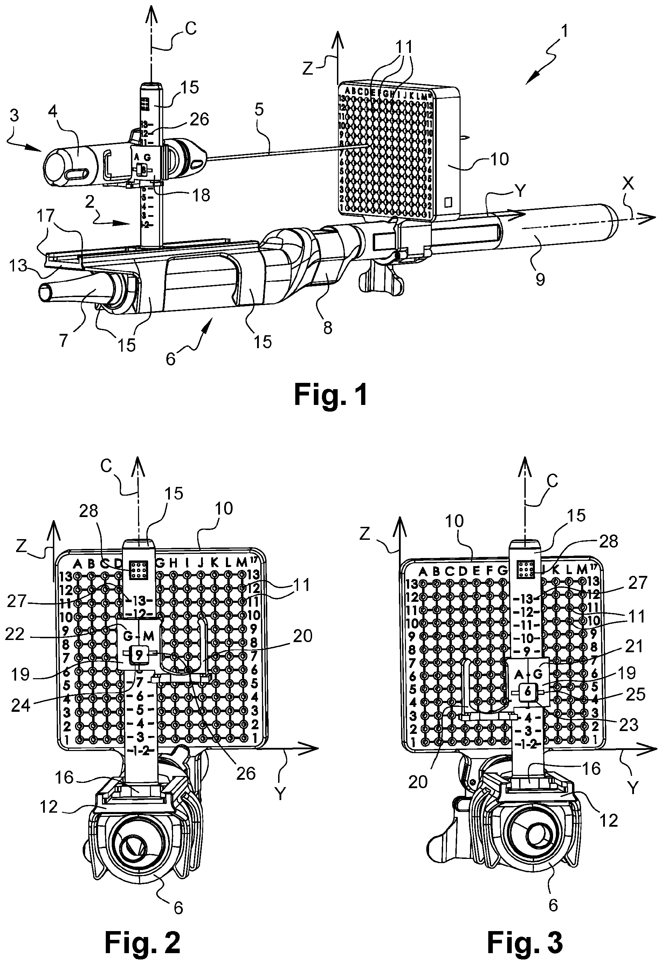

En référence aux figures 1 à 3, selon un premier mode de réalisation de l'invention, l'installation, généralement désignée en 1, comporte un dispositif de maintien 2 qui est associé à un instrument médical. L'instrument médical est ici un applicateur 3 pour un traitement par micro-ondes d'au moins une zone d'une prostate d'un patient et ce par une approche transpérinéale.

Cette application n'est bien entendu pas limitative et on pourra utiliser l'installation 1 comme le dispositif de maintien 2 en association avec différents types d'actes médicaux (biopsie, curiethérapie, cryothérapie, thérapie laser ...) ; différentes approches (transpérinéale, transrectale ...) et différentes cibles (prostate, rein, foie, utérus ...) .

L'installation 1 comme le dispositif de maintien 2 pourront ainsi être associés à d'autres instruments que l'applicateur 3 comme par exemple un porte-aiguille à biopsie.

Afin de pouvoir effectuer un traitement ciblé, l'applicateur 3 comporte notamment un manche 4 à partir duquel s'étend une aiguille 5 de traitement. Le manche 4 comporte par ailleurs un connecteur pour son rattachement à un câble stérile. Un tel applicateur 3 est bien connu de l'homme du métier et ne sera pas davantage détaillé ici.

L'installation 1 comporte par ailleurs un appareil 6 de contrôle s'étendant généralement longitudinalement selon un axe Z afin de faciliter son insertion dans le rectum du patient.

De préférence, l'appareil 6 comporte un support 7, s'étendant parallèlement à l'axe Z, qui est destiné à être saisi au niveau de son extrémité proximale par un opérateur et qui est prolongé à son extrémité distale par un décrochement 8 de l'appareil 2. Ledit décrochement 8 est lui-même prolongé par une sonde 9 d'imagerie médicale de l'appareil 2 pour permettre la génération d'images en trois dimensions de la prostate et de son environnement, sonde 9 s'étendant également selon l'axe Z. De la sorte, l'appareil 2 est plus aisément manipulable par l'opérateur du fait du décrochement 8.

Pour faciliter le travail de l'opérateur, l'installation 1 peut comprendre un système de co-manipulation de l'appareil 6, lié à l'extrémité proximale de l'appareil 6. Le système de co-manipulation est par exemple un robot, un bras articulé ...

La sonde 9 est ici une sonde échographique qui est donc destinée à être introduire dans le rectum du patient. Cette application n'est bien entendu pas limitative et on pourra utiliser le dispositif de maintien 2 avec d'autres types d'imageries i.e. d'autres types de sondes. L'installation 1 comporte de préférence un guide-aiguille 10 solidaire de l'appareil 6. Plus précisément ici, le guide-aiguille 10 est porté par la sonde 9. Le guide-aiguille 10 est donc agencé sur l'appareil 6 en aval du décrochement 8 (selon un sens allant du support 7 à la sonde 9).

De préférence, le guide-aiguille 10 est monté coulissant sur la sonde 9 afin de pouvoir modifier sa position vis-à-vis du patient.

Le guide-aiguille 10 comporte par ailleurs une grille qui s'étend ici dans un plan sensiblement de normale l'axe Z. La grille comporte des orifices 11 (une partie seulement est référencée ici), tous parallèles les uns aux autres, et à l'axe Z. Les différents orifices 11 sont par ailleurs agencés en colonnes sur la grille parallèlement à un axe X, orthogonal à l'axe Z et agencés en lignes sur la grille parallèlement à un axe Y, orthogonal à l'axe X et à l'axe Z.

L'aiguille 5 peut ainsi être insérée dans le guide-aiguille 10 à une hauteur différente (selon l'axe X) ce qui permet de définir la hauteur à laquelle l'aiguille 5 vient s'enfoncer dans le périnée mais également à une largeur différente dans le guide-aiguille 10 (selon l'axe Y) ce qui permet de définir la distance à laquelle l'aiguille 5 vient s'enfoncer dans le périnée. On peut ainsi guider l'aiguille 5 tout en ayant une liberté selon deux degrés de liberté sur la zone d'insertion de l'aiguille 5 dans le périnée.

Préférentiellement la grille elle-même comporte des repères graphiques (graduations, caractères alphanumériques ...) pour identifier les différentes colonnes d'orifices de la grille et des repères graphiques différents pour identifier les différentes lignes d'orifices de la grille. Optionnellement, la grille comporte des lettres pour identifier chaque colonne et des chiffres pour identifier chaque ligne.

On va à présent s'attacher à décrire le dispositif de maintien 2.

Le dispositif de maintien 2 comporte tout d'abord une base 12 qui est portée par l'appareil 6. Plus précisément ici, la base 12 est portée par le support 7. La base 12 se situe donc en amont du décrochement 8.

La base 12 comporte un plateau 13 s'étendant sensiblement sur toute la longueur du support 7 parallèlement à l'axe Z.

Par ailleurs, la base 12 comporte des moyens de fixation temporaire à l'appareil 6 comme des moyens d'encliquetage. La base 12 comporte de préférence une première paire de bras 14 et une deuxième paire de bras 15, les deux paires de bras étant agencées à des extrémités longitudinales opposées du plateau 13 pour l'encliquetage de la base 12 au support 7.

La base 12 est donc ainsi directement portée par le support 7 sans élément intermédiaire.

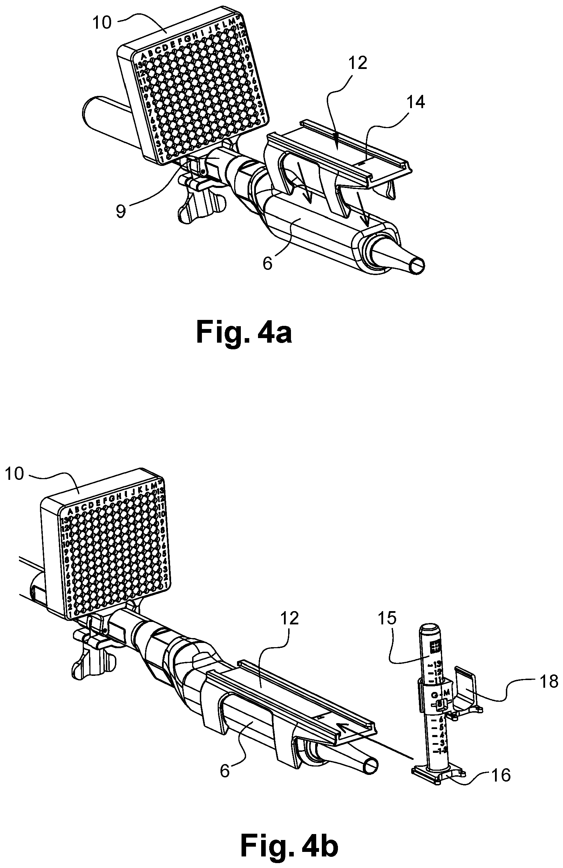

De préférence (comme plus visible à la figure 4a), la base 12 comporte un signe de repérage 14 permettant à l'opérateur de placer plus rapidement de manière correcte la base 12 sur le support 7. Par exemple le signe de repérage 14 est un élément graphique (ligne, flèche ...) symbolisant l'une des extrémités longitudinales du support 7, l'opérateur devant agencer la base 12 de manière que le signe de repérage 14 soit sensiblement à l'aplomb de ladite extrémité longitudinale.

Le dispositif de maintien 2 comporte en outre une structure porteuse comprenant ici une colonne 15 montée mobile sur la base 12 de manière à pouvoir se déplacer sur la base 12 entre une première position et une deuxième position. La première position est ici dite position de travail soit une position dans laquelle la colonne 15 est à niveau de l'applicateur 3, et plus exactement de son manche 4. La deuxième position est ici dite de manoeuvre soit une position dans laquelle la colonne 15 est éloignée de l'applicateur 3.

La colonne 15 est ici (hors extrémité inférieure) de section circulaire.

Optionnellement, la colonne 15 est montée coulissante sur la base 12 entre ces deux positions. A cet effet, l'extrémité inférieure de la colonne 15 est conformée en un socle 16 apte à coulisser dans une paire de rails 17 correspondants de la base 12. La colonne 15 est donc ainsi directement portée par la base 12 sans élément intermédiaire.

La colonne 15, lorsqu'elle est montée sur la base 12, s'étend en conséquence sensiblement à la normale de la base 12. Dans le cas présent, la colonne 15 s'étend ainsi selon un axe C qui, en service, est parallèle à l'axe X, la colonne coulissant alors parallèlement à l'axe Z sur le support 7.

Plus précisément ici, les rails 17 s'étendent longitudinalement parallèlement à l'axe Z sur une face supérieure du plateau 13 de sorte à border longitudinalement les deux côtés de ladite face.

De préférence, les rails 17 s'étendent au moins jusqu'à l'une des extrémités longitudinales du plateau 13. Dans le cas présent, les rails 17 s'étendent jusqu'aux deux extrémités du plateau 13 de sorte à déboucher à leurs deux extrémités. Il est ainsi possible de séparément temporairement la colonne 15 du plateau 13 en faisant coulisser la colonne 15 jusqu'à l'une ou l'autre des extrémités longitudinales du plateau 13.

Ceci facilite le montage du dispositif de maintien 2 et sa manipulation.

De préférence alors, le socle 16 est conformé de manière que la colonne 15 ne puisse être insérée dans la base 12 que selon une seule orientation possible vis-à-vis de la base 12 ou que selon deux orientations possibles. Par exemple le socle 16 n'est pas conformé en un carré mais un rectangle de manière que le socle 16 ne puisse pas être introduit dans les rails 17 par ses deux plus grands côtés et/ou est conformé en un carré ne présentant des languettes aptes à coopérer avec les rails 17 que sur de seulement de ses côtés.

De préférence, la structure porteuse comporte une bague 18 montée mobile sur la colonne 15. La bague 18 est directement portée par la colonne 15 sans élément intermédiaire.

La bague 18 est ici montée coulissante sur la colonne 15.

De façon préférée, la bague 18 est en outre agencée sur la colonne 15 de manière à pouvoir pivoter autour de l'axe C lorsque le dispositif de maintien 2 est en place sur l'appareil 6.

La bague 18 peut ici pivoter à 360 degrés autour de la colonne 15 selon l'axe C.

Typiquement, la bague 18 comporte un tube 19 de rattachement à la colonne 15. Le tube 19 s'étend selon une direction longitudinale et présente un orifice central coaxial à ladite direction, orifice traversant le tube 19 de part en part. Lorsque le tube 19 est monté sur la colonne 15, la direction longitudinale du tube est confondue avec la direction générale de la colonne 15 soit l'axe C. L'orifice présente une section similaire à celle de la colonne 15 pour autoriser le coulissement et la rotation de la bague 18 sur la colonne 15.

Par ailleurs la bague 18 comporte des moyens de fixation temporaire de l'applicateur 3 à la bague 18, comme des moyens d'encliquetage 20.

Les moyens d'encliquetage 20 sont portés par le tube 19 de sorte à s'étendre à côté du tube 19. Les moyens d'encliquetage 20 sont ainsi agencés en service à côté de la colonne 15 et non sur la colonne 15.

Les moyens d'encliquetage 20 comportent de préférence une platine à partir de laquelle s'étendent latéralement deux bras pour l'encliquetage de l'applicateur 3 à la bague 18. L'applicateur 3 est ainsi directement porté par la bague 18 sans élément intermédiaire.

Un des bras est ici fixé au tube 19. De préférence, le premier bras est fixé au tube 19 de sorte que le deuxième bras s'étende sensiblement parallèlement à l'axe C, et à distance de la colonne 15, lorsque la bague 18 est en place sur la colonne 15.

Optionnellement le tube 19 et les moyens d'encliquetage 20 sont d'une seule pièce.

Préférentiellement, la bague 18 comporte elle-même des repères graphiques associés aux repères graphiques des colonnes du guide-aiguille 10 dans le but de permettre d'orienter correctement le dispositif de maintien 2 vis-à-vis desdites colonnes du guide-aiguille 10.

Dans le cas présent, et comme plus visible aux figures 2 et 3, la bague 18 comporte un premier repère graphique 21 sur un premier secteur angulaire du tube 19 et un deuxième repère graphique 22 sur un deuxième secteur angulaire du tube 19 opposé au premier secteur angulaire.

Le premier repère graphique 21 renvoie à la première moitié des repères graphiques des colonnes (ici en indiquant la première lettre et la dernière lettre de cette première moitié) et le deuxième repère graphique 22 renvoie à la deuxième moitié des repères graphiques des colonnes (ici en indiquant la première lettre et la deuxième lettre de cette deuxième moitié).

De la sorte, une fois le dispositif de maintien 2 en place dans l'installation 1, selon que l'on place la bague 18 de manière que l'opérateur voit le premier repère graphique 21 ou le deuxième repère graphique 22, la bague 18 est tournée de 360 degrés ; et donc les moyens d'encliquetage 20 également. Le dispositif de maintien 2 est ainsi agencé de sorte que les moyens d'encliquetage 20 se trouvent soit à droite soit à gauche de la colonne 15 selon que le premier repère graphique 21 ou le deuxième repère graphique 22 fasse face à l'opérateur (bien entendu on se place dans la position de service usuelle dans laquelle l'opérateur tient le support 7 et regarde le guide-aiguille 10). Bien entendu les moyens d'encliquetage 20 de la bague 18 sont alors conformés de manière à pouvoir accueillir l'applicateur 3 que les moyens d'encliquetage 20 soient à gauche ou à droite de la colonne 15.

En service, selon l'orientation de la bague 18 vis-à-vis de la colonne 15, l'applicateur 3 sera donc à droite ou à gauche de la colonne 15.

Le dispositif de maintien 2 permet donc de faciliter la tâche de l'opérateur en portant l'applicateur 3 mais également en l'aidant à positionner le dispositif de maintien 2 vis-à-vis de l'applicateur 3 de manière relativement fine.

Préférentiellement, le tube 19 est pourvu de deux fenêtres 23, 24 à travers lesquelles il est possible de visualiser la colonne 15, les deux fenêtres étant ménagées sur des secteurs annulaires opposés du tube 19. Au-dessus de chaque fenêtre 23, 24 est agencé l'un des repères graphiques 21, 22 de la bague 18.

La colonne 15 comporte par ailleurs des repères graphiques le long de son axe C en relation avec ceux des lignes du guide-aiguille 10. Les repères graphiques sont donc ici une succession de chiffres.

De la sorte, lorsque le tube 19 est déplacé le long de la colonne 15, il est possible de visualiser un repère graphique différent de la colonne 15 à travers l'une des fenêtres 23, 24.

Dans le cas présent, la colonne 15 comporte autant de repères graphiques régulièrement répartis sur sa hauteur qu'il y a de lignes sur le guide-aiguille 10, à chaque repère graphique correspondant une des lignes du guide-aiguilles 10, à l'exception des deux lignes les plus basses qui sont regroupées sur un seul repère graphique.

Optionnellement, le tube 19 comporte au moins un sigle de centrage 25, 26 associé à chaque fenêtre afin de placer le tube 19 de manière que le repère graphique visé de la colonne 15 soit bien placé au centre de la fenêtre 23, 24 considérée par l'opérateur. Chaque fenêtre 23, 24 est ici associée à deux traits appartenant à la même ligne horizontale médiane traversant ladite fenêtre 23, 24, les traits étant agencés de part et d'autre de ladite fenêtre 23, 24. D'autres sigles de centrage peuvent être employés comme un trait vertical, ou une ou des flèches, une ou des étoiles ...

De préférence, chaque repère graphique de la colonne 15 est également associé à au moins un sigle de centrage 27 (une partie seulement étant référencée ici) afin de faciliter encore davantage le centrage de la fenêtre 23, 24 considérée sur le repère graphique de la colonne 15 visé. Typiquement chaque chiffre de la colonne 15 est entouré de deux traits appartenant à une même ligne horizontale. On choisit de préférence des fenêtres 23, 24 de dimensions suffisamment importantes de manière qu'au moins une partie des sigles de centrage 27 du repère visé de la colonne 15 soient visibles à travers la fenêtre 23, 24 considérée.

Ainsi, l'opérateur peut plus facilement centrer la fenêtre 23, 24 sur le repère graphique visé.

Le dispositif de maintien 2 permet donc de faciliter la tâche de l'opérateur en portant l'applicateur 3 mais également en l'aidant à positionner de manière fine, à la bonne hauteur, le dispositif de maintien 2 vis-à-vis de l'applicateur 3.

Nous allons à présent décrire la manière dont le dispositif de maintien 2 est agencé dans l'installation 1 en référence aux figures 4a à 4f.

En référence à la figure 4a, la base 12 est encliquetée sur le support 7 puis la sonde 9 est insérée dans le rectum et positionnée selon une orientation et une position visées particulières en vue d'un traitement.

En référence à la figure 4b, on vient alors monter la colonne 15 sur la base 12 en insérant le socle 16 dans les rails 17. On positionne également la bague 18 sur la colonne 15.

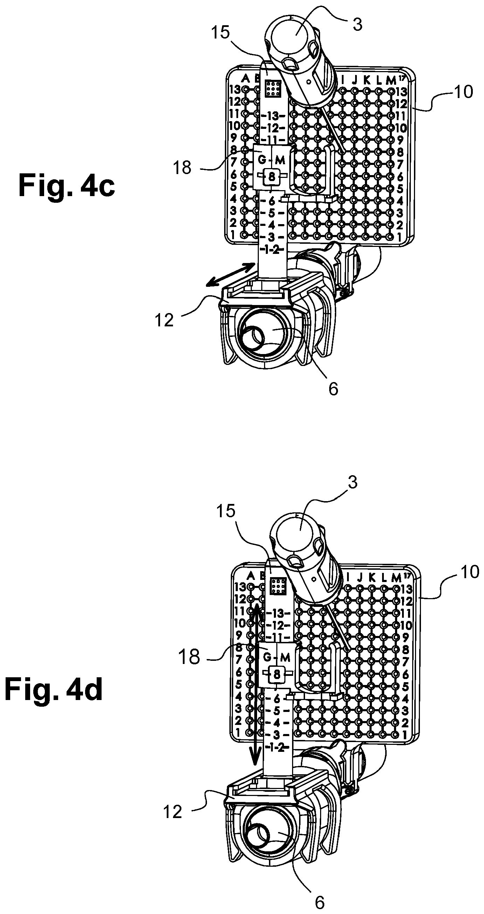

En référence à la figure 4c, l'opérateur vient ensuite positionner l'applicateur 3 selon l'orientation et la position visées pour le traitement en s'aidant notamment du guide-aiguille.

Une fois l'aiguille 5 positionnée, il devient nécessaire de maintenir en place l'applicateur 3. A cet effet, l'opérateur oriente la bague 18 sur le côté droit si l'applicateur 3 est dans la partie droite du guide-aiguille 10 ou sur le côté gauche si l'applicateur 3 est dans la partie gauche du guide-aiguille 10. On voit bien ici l'intérêt des repères graphiques du dispositif de maintien 2 associés à ceux du guide-aiguille 10 qui facilitent le travail de l'opérateur.

L'opérateur coulisse ensuite la colonne 15 sur la base 12 jusqu'au manche de l'applicateur 3.

En référence à la figure 4d, l'opérateur ajuste alors la hauteur de la bague 18 afin de se rapprocher de l'applicateur 3. Là encore, on voit bien l'intérêt des repères graphiques du dispositif de maintien 2.

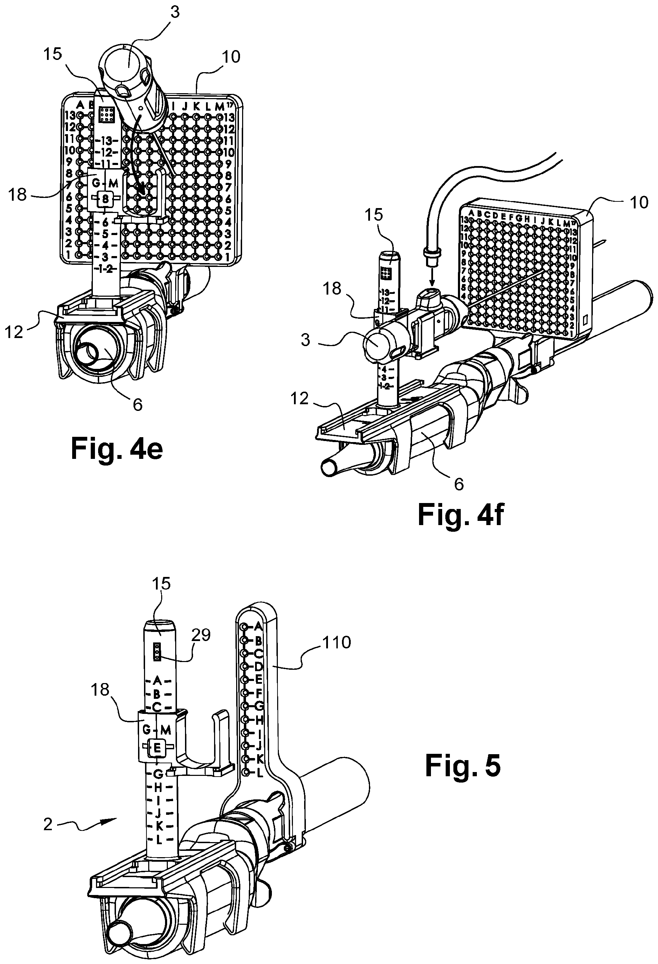

En référence à la figure 4e, l'opérateur vient encliqueter le manche 4 dans la bague 18. L'applicateur 3 est ainsi maintenu en place par le dispositif de maintien 2.

En référence à la figure 4f, l'opérateur connecte l'applicateur 3 à un câble stérile pour débuter le traitement.

Le dispositif de maintien 2 ainsi décrit permet notamment bien que non exclusivement :
- de maintenir fixe la position de l'applicateur 3 et ce pendant les minutes ou dizaines de minutes que va durer le traitement,
- d'assurer un contrôle précis de la position de l'aiguille 5 en profondeur, le dispositif de maintien 2 empêchant un coulissement involontaire de l'applicateur 3 parallèlement à l'axe Z,
- de pouvoir en outre ajuster précisément la profondeur à laquelle l'aiguille 5 est insérée dans la prostate grâce au déplacement possible de l'applicateur 3 selon l'axe Z, l'opérateur pouvant ainsi contrôler avec précision l'insertion en profondeur (selon l'axe Z) de l'aiguille 5 en avançant sereinement l'applicateur 3 petit à petit (voir millimètre par millimètre) selon l'axe Z jusqu'à atteindre une cible visée,
- de laisser la possibilité à l'opérateur d'accéder malgré tout à l'ensemble du guide-aiguille 10 et donc à l'organe du fait notamment du montage ultérieur du dispositif de maintien 2 sur le support 6,
- d'être d'un encombrement réduit limitant ainsi un risque de gêne de l'opérateur durant le traitement,
- de pouvoir être placé mais aussi retiré de manière simple et rapide,
- d'être de construction simplifiée permettant une usinabilité aisée du dispositif.

On remarque que le dispositif de maintien 2 permet de s'approcher finalement de l'applicateur 3 grâce à ses deux translations et sa rotation.

Dans le cas présent, on profite avantageusement de la flexibilité de l'aiguille 5 pour ne permettre une orientation de la bague 18 que du côté gauche ou du côté droit de la colonne 15 (en place d'une translation complète parallèlement à l'axe Y). Comme indiqué ci-dessous on pourra toutefois prévoir que le dispositif de maintien 2 autorise une translation de la bague 18 selon l'axe Y pour s'approcher de manière encore plus fine de l'applicateur 3.

Par ailleurs la construction simplifiée du dispositif permet de pouvoir construire très simplement le dispositif par exemple par moulage par injection ou bien par imprimante en trois dimensions. Dans le cas présent, la base 12, la colonne 15 et la bague 18 sont chacune moulées par injection.

En référence à la figure 5, un deuxième mode de réalisation va être à présent décrit.

A la différence du premier mode de réalisation, le guide-aiguille 110 ne se présente pas sous la forme d'une grille mais d'un poteau. En conséquence, le guide-aiguille 110 ne comporte qu'une seule colonne d'orifices s'étendant selon l'axe X.

Dans ce cas de figure, le dispositif de maintien peut alors être conformé différemment que dans le premier mode de réalisation avec une bague qui coulisse le long de la colonne sans toutefois pivoter autour de la colonne.

Préférentiellement, on préfère utiliser malgré tout le même dispositif de maintien 2 que dans le premier mode de réalisation.

De façon avantageuse, dans le cas où le guide-aiguille 110 n'utilise pas les mêmes repères graphiques que le guide-aiguille 10 du premier mode de réalisation, la colonne 15 est conformée pour présenter une première succession de repères graphiques correspondant au premier guide-aiguille 10 et une deuxième succession de repères graphiques correspondant au deuxième guide-aiguille 110, les deux successions de repère graphiques étant agencées sur des secteurs angulaires opposés de la colonne 15 à l'aplomb respectivement de l'un des deux côtés du socle 16 par lequel celui-ci peut être inséré dans les rails 17.

De la sorte, selon que la colonne 15 est insérée par un premier côté du socle 16 dans les rails 17 ou par un deuxième côté, l'opérateur ne visualise pas la même succession de repères graphiques.

Un même dispositif de maintien peut en conséquence être utilisé avec deux guides-aiguilles différents.

Optionnellement, la colonne 15 comporte un premier élément de repérage 28 de la première succession de repères graphiques et un deuxième élément de repérage 29 de la deuxième succession de repères graphiques afin que l'opérateur puisse différencier plus facilement les deux successions de repères graphiques.

Par exemple le premier élément de repérage 28 reproduit schématiquement une grille et le deuxième élément de repérage 29 reproduit schématique un poteau afin que l'opérateur puisse aisément associer le côté de la colonne 15 associé au bon guide-aiguille.

En référence à la figure 6, un troisième mode de réalisation va être à présent décrit.

A la différence du premier mode de réalisation, le guide-aiguille 210 ne se présente pas sous la forme d'une grille mais d'un tunnel. En conséquence, le guide-aiguille 210 ne comporte qu'un seul orifice.

Dans ce cas de figure, le dispositif de maintien peut alors être conformé différemment que dans le premier mode de réalisation de manière que la structure porteuse ne comporte pas de colonne mais seulement une bague montée mobile sur la base.

Dans le cas présent, la base 12 est identique à celles des autres modes de réalisation.

De préférence, la bague 18 est également identique à celles des autres modes de réalisation.

Ainsi, afin que la bague 18 coulisse sur la base 12 entre la position de travail dans laquelle la bague 18 est à niveau de l'applicateur 3, et plus exactement de son manche 4, et une position de manoeuvre dans laquelle la bague 18 est éloignée de l'applicateur 3, l'extrémité inférieure de la bague 18 est conformée en un socle 40 (plus visible aux figures 4b et 5) apte à coulisser dans la paire de rails 17 correspondants de la base 12. La bague 18 est donc ainsi directement portée par la base 12 sans élément intermédiaire.

Le socle 40 est ici agencé en partie basse des moyens d'encliquetage 20 et plus particulièrement de sa platine.

Indifféremment, le socle 40 est conformé de manière que la bague 18 puisse être insérée dans la base 12 selon une seule orientation possible vis-à-vis de la base 12 ou selon deux orientations possibles. Par exemple le socle 40 n'est pas conformé en un carré mais un rectangle de manière que le socle 40 ne puisse pas être introduit dans les rails 17 par ses deux plus grands côtés et/ou est conformé en un carré ne présentant des languettes aptes à coopérer avec les rails 17 que sur de seulement de ses côtés.

De façon avantageuse, en retirant la colonne 15 de la structure porteuse et en montant la bague 18 directement sur la base 12, le même dispositif de maintien peut ainsi être utilisé avec trois types de guide-aiguille différent.

Bien entendu l'invention n'est pas limitée aux modes de réalisation décrits et on peut y apporter des variantes sans sortir du cadre de l'invention tel que défini par les revendications.

En particulier, le dispositif de maintien pourra être utilisé pour d'autres applications que celles décrites. Notamment, le dispositif de maintien pourra être utilisé seul et non en association avec un guide-aiguille.

Par ailleurs, l'installation pourra comporter un nombre différent d'instruments et/ou comporter des instruments différents de ce qui a été indiqué en fonction des applications visées. En fonction de l'application à laquelle sera destiné le dispositif de maintien, l'instrument pourra ainsi changer. L'instrument pourra être longiforme ou non. L'instrument pourra ainsi être un instrument de biopsie, de traitement, un instrument chirurgical, une aiguille, un applicateur, une fibre ... Si l'installation comporte au moins deux instruments, le dispositif pourra ainsi comporter au moins deux applicateurs de cryothérapie, au moins deux fibres laser ... On pourra alors avoir deux dispositifs de maintien, un pour chaque instrument, ou bien avoir un même dispositif de maintien portant les deux instruments.

Le dispositif de maintien pourra être directement porté par la sonde d'imagerie médicale.

Bien qu'ici la structure porteuse soit mobile sur la base selon un unique mouvement de translation, la structure porteuse pourra être montée sur la base selon un autre mouvement comme une ou plusieurs translations et/ou une ou plusieurs rotations.

La structure porteuse pourra prendre une autre forme que celle décrite. Par exemple la structure porteuse pourra comporter une colonne présentant (hors socle d'attache à la base) une section ovale, carré, rectangulaire ... La structure porteuse pourra ne pas comporter de colonne. De la même manière, la structure porteuse pourra ne pas comporter de bague. La bague pourra être montée différemment sur la colonne. Par exemple la bague pourra n'être montée que pivotante selon la direction générale de la colonne porteuse ou n'être montée que coulissante le long de la colonne. La bague pourra être liée par une autre cinématique que celle décrite à la colonne. Par exemple la bague pourra être liée par une liaison rotule à la colonne ou bien par deux liaisons pivots ou bien par deux liaisons glissières. On pourra ainsi avoir une bague montée pivotante sur la colonne selon l'axe de colonne et selon un axe parallèle à l'axe X. On pourra avoir une bague montée en translation sur la colonne selon l'axe de colonne et selon un axe parallèle à l'axe X. La bague pourra avoir un nombre différent de fenêtres que ce qui a été indiqué et par exemple une seule fenêtre.

La structure porteuse pourra être conformée de manière que ses éléments soient tous fixes entre eux.

On pourra ainsi avoir un dispositif de maintien comprenant une structure porteuse montée mobile seulement en translation sur la base selon un seul axe de manière qu'une fois maintenu sur la structure porteuse l'instrument ne puisse être déplacé que selon ledit axe par un mouvement volontaire de l'opérateur. Ceci permettra ainsi de contrôler au moins la profondeur à laquelle l'instrument est positionné vis-à-vis du patient. Un tel dispositif est illustré à la figure 6.

En cas de rotation d'un élément par rapport à un autre au sein du dispositif de maintien, la rotation pourra être complète (soit de 360 degrés) ou partielle.

Les différentes étapes de montage du dispositif de maintien sur l'appareil pourront être différentes de ce qui a été indiqué. En particulier on pourra d'abord orienter grossièrement le dispositif de maintien vis-à-vis de l'instrument puis affiner au fur et à mesure le positionnement du dispositif de maintien vis-à-vis de l'instrument. En outre, bien qu'ici l'instrument soit déjà en place lorsqu'on le rattache au dispositif de maintien, l'instrument pourra n'être que partiellement en place ou non en place. On s'aidera alors du dispositif de maintien pour manipuler l'instrument grâce aux différentes orientations autorisées par les mouvements des éléments du dispositif de maintien entre eux.

Bien qu'ici les différents éléments du dispositif de maintien soient mobiles entre eux de manière manuelle, un, plusieurs ou tous les mouvements pourront être motorisés.

Bien qu'ici le simple frottement entre les différents éléments du dispositif de maintien suffise pour maintenir en place lesdits éléments relativement entre eux, le dispositif de maintien pourra comporter des moyens de fixation temporaires d'au moins deux éléments du dispositif de maintien entre eux (par exemple via des pions, des vis, des brides, par encliquetage ...)

De la même manière, bien que les moyens de fixation décrits soit des moyens d'encliquetage, les moyens de fixation pourront être différents (par exemple des pions, des vis, des brides ...) .

## Revendications

1. Dispositif de maintien d'un instrument médical, le dispositif comprenant :
- une base (12) destinée à être portée par un appareil (6) comprenant une sonde (9) d'imagerie médicale associée à l'instrument médical (3), et
- une structure porteuse destinée à porter l'instrument médical et montée mobile sur la base de manière à pouvoir se déplacer sur la base entre une première position et une deuxième position,
le dispositif étant apte à maintenir comme instrument médical un applicateur comprenant un manche (4) à partir duquel s'étend une aiguille (5) de traitement, la structure porteuse comprenant à cet effet une bague destinée à porter le manche de l'instrument médical.

2. Dispositif selon la revendication 1, dans lequel la bague n'est pas portée par un porte-aiguille du dispositif.

3. Dispositif selon la revendication 1 ou selon la revendication 2, dans lequel la structure porteuse est montée coulissante sur la base (12).

4. Dispositif selon l'une des revendications 1 à 3, dans lequel la structure porteuse comporte une colonne (15) montée mobile sur la base et une bague (18) montée mobile sur la colonne.

5. Dispositif selon la revendication 4, dans lequel la bague (18) est montée coulissante sur la colonne (15).

6. Dispositif selon la revendication 4 ou la revendication 5, dans lequel la bague (18) est montée pivotante sur la colonne.

7. Dispositif selon l'une des revendications 1 à 2 ou 4 à 6, dans lequel la bague (18) comporte des moyens de fixation temporaire de l'instrument au dispositif.

8. Dispositif selon l'une des revendications 4 à 7, dans lequel la bague (18) comporte au moins une fenêtre (23, 24) pour visualiser la colonne.

9. Dispositif selon l'une des revendications 4 à 8, dans lequel la bague (18) comporte au moins un sigle de repérage de l'orientation relative de la bague vis-à-vis de la colonne (15).

10. Dispositif selon l'une des revendications 4 à 9, dans lequel la colonne (15) comporte au moins une succession de repères graphiques.

11. Dispositif selon l'une des revendications 4 à 10, dans lequel la bague (18) est conformé pour pouvoir être montée mobile sur la base (12) sans la colonne (15).

12. Dispositif selon l'une des revendications précédentes, dans lequel la structure porteuse peut être montée sur la base (12) selon au moins deux sens d'introduction différents.

13. Dispositif selon la revendication 12, dans lequel la structure porteuse comporte un élément de repérage pour chaque sens d'introduction possible de la structure porteuse sur la base (12).

14. Dispositif selon l'une des revendications précédentes, dans lequel la base (12) comporte au moins un signe de repérage pour symboliser où la base doit être montée en service sur l'appareil.

15. Installation comprenant un dispositif selon l'une des revendications précédentes et un guide-aiguille (10 ; 110 ; 210).

16. Installation selon la revendication 15, dans laquelle le guide-aiguille est une grille (10).

17. Installation selon la revendication 15 ou la revendication 16, comportant en outre l'appareil qui comporte une sonde échographique.

18. Installation selon l'une des revendications 15 à 17, comprenant en outre l'instrument médical qui n'est pas un guide-aiguille.

## Patentansprüche

1. Vorrichtung zum Halten eines medizinischen Instruments, wobei die Vorrichtung umfasst:
- eine Basis (12), die dazu bestimmt ist, von einem Gerät (6) getragen zu werden, das eine Sonde (9) zur medizinischen Bildgebung umfasst, die mit dem medizinischen Instrument (3) verknüpft ist, und
- eine tragende Struktur, die dazu bestimmt ist, das medizinische Instrument zu tragen und beweglich so auf der Basis montiert ist, dass sie sich auf der Basis zwischen einer ersten Position und einer zweiten Position verschieben kann,
wobei die Vorrichtung geeignet ist, als medizinisches Instrument einen Applikator zu halten, der einen Griff (4) umfasst, aus dem sich eine Behandlungsnadel (5) erstreckt, wobei die tragende Struktur zu diesem Zweck einen Ring umfasst, der dazu bestimmt ist, den Griff des medizinischen Instruments zu tragen.

2. Vorrichtung nach Anspruch 1, bei der der Ring nicht von einem Nadelhalter der Vorrichtung getragen wird.

3. Vorrichtung nach Anspruch 1 oder nach Anspruch 2, bei der die tragende Struktur verschiebbar auf der Basis (12) gelagert ist.

4. Vorrichtung nach einem der Ansprüche 1 bis 3, bei der die tragende Struktur eine Säule (15) umfasst, die an der Basis beweglich gelagert ist, und einen Ring (18), der beweglich an der Säule gelagert ist.

5. Vorrichtung nach Anspruch 4, bei der der Ring (18) verschiebbar an der Säule (15) gelagert ist.

6. Vorrichtung nach Anspruch 4 oder Anspruch 5, bei der der Ring (18) schwenkbar an der Säule gelagert ist.

7. Vorrichtung nach einem der Ansprüche 1 bis 2 oder 4 bis 6, bei der der Ring (18) Mittel zur temporären Befestigung des Instruments an der Vorrichtung umfasst.

8. Vorrichtung nach einem der Ansprüche 4 bis 7, bei der der Ring (18) mindestens ein Fenster (23, 24) umfasst, um die Säule zu visualisieren.

9. Vorrichtung nach einem der Ansprüche 4 bis 8, bei der der Ring (18) mindestens ein Markierungszeichen zur relativen Ausrichtung des Rings gegenüber der Säule (15) umfasst.

10. Vorrichtung nach einem der Ansprüche 4 bis 9, bei der die Säule (15) mindestens eine Folge von graphischen Markierungen umfasst.

11. Vorrichtung nach einem der Ansprüche 4 bis 10, bei der der Ring (18) derart geformt ist, dass er ohne die Säule (15) beweglich an der Basis (12) gelagert werden kann.

12. Vorrichtung nach einem der vorhergehenden Ansprüche, bei der die tragende Struktur an der Basis (12) in mindestens zwei unterschiedlichen Einführungsrichtungen montiert werden kann.

13. Vorrichtung nach Anspruch 12, bei der die tragende Struktur ein Markierungselement für jede mögliche Einführungsrichtung der tragenden Struktur an der Basis (12) umfasst.

14. Vorrichtung nach einem der vorhergehenden Ansprüche, bei der die Basis (12) mindestens ein Markierungszeichen umfasst, um zu symbolisieren, wo die Basis im Gebrauch an dem Gerät montiert werden soll.

15. Anlage, umfassend eine Vorrichtung nach einem der vorhergehenden Ansprüche und eine Nadelführung (10; 110; 210).

16. Anlage nach Anspruch 15, bei der die Nadelführung ein Gitter (10) ist.

17. Anlage nach Anspruch 15 oder Anspruch 16, ferner umfassend das Gerät, das eine Ultraschallsonde umfasst.

18. Anlage nach einem der Ansprüche 15 bis 17, ferner umfassend ein medizinisches Instrument, das keine Nadelführung ist.

## Claims

1. A holder device for holding a medical instrument, the device comprising:
· a base (12) that is to be carried by an apparatus (6) comprising a medical imaging probe (9) associated with the medical instrument (3); and
· a carrier structure for carrying the medical instrument and movably mounted on the base so as to be capable of moving on the base between a first position and a second position,
wherein the device is suitable for holding a medical instrument in the form of an applicator comprising a handle (4) from which there extends a treatment needle (5), the carrier structure including, for this purpose, a ring for carrying the handle of the medical instrument.

2. A device according to claim 1, wherein the ring is not carried by a needle-carrier of the device.

3. A device according to claim 1 or according to claim 2, wherein the carrier structure is slidably mounted on the base (12).

4. A device according to any one of claims 1 to 3, wherein the carrier structure comprises a column (15) movably mounted on the base and a ring (18) movably mounted on the column.

5. A device according to claim 4, wherein the ring (18) is slidably mounted on the column (15).

6. A device according to claim 4 or claim 5, wherein the ring (18) is pivotally mounted on the column.

7. A device according to any one of claims 1 to 2 or 4 to 6, wherein the ring (18) includes means for fastening the instrument temporarily to the device.

8. A device according to any one of claims4 to 7, wherein the ring (18) includes at least one window (23, 24) for viewing the column.

9. A device according to any one of claims 4 to 8, wherein the ring (18) includes at least one position-marker symbol for identifying the orientation of the ring relative to the column (15).

10. A device according to any one of claims 4 to 9, wherein the column (15) includes at least one succession of graphical marks.

11. A device according to any one of claims 4 to 10, wherein the ring (18) is shaped to be capable of being movably mounted on the base (12) without the column (15).

12. A device according to any preceding claim, wherein the carrier structure can be mounted on the base (12) in at least two different engagement directions.

13. A device according to claim 12, wherein the carrier structure includes a direction-marker element for each possible direction for engaging the carrier structure on the base (12).

14. A device according to any preceding claim, wherein the base (12) includes at least one position-marker sign for symbolizing where the base is to be mounted in service on the apparatus.

15. An installation comprising both a device according to any preceding claim, and a needle-guide (10; 110; 210).

16. An installation according to claim 15, wherein the needle-guide is a grid (10).

17. An installation according to claim 15 or claim 16, further comprising the apparatus that includes an ultrasound probe.

18. An installation according to any one of claims 15 to 17, further comprising the medical instrument that is not a needle-guide.
